# EUROPÄISCHE PATENTSCHRIFT

(11) **EP 0 983 202 B1**
(45) Veröffentlichungstag und Bekanntmachung des Hinweises auf die Patenterteilung: **22.01.2003**
(21) Anmeldenummer: 98921451.5
(22) Anmeldetag: 14.04.1998
(51) Int. Cl.: B65D 75/52, B65D 75/58, A61F 6/00

(54) **KONDOMVERPACKUNG ZUM EINHÄNDIGEN ÖFFNEN**
CONDOM PACKAGE THAT CAN BE OPENED WITH ONE HAND
EMBALLAGE POUR PRESERVATIFS POUVANT ETRE OUVERT D'UNE SEULE MAIN

(30) Priorität: 15.04.1997 DE 29707252 U
(43) Veröffentlichungstag der Anmeldung: 08.03.2000
(73) Patentinhaber: Flock, Romanus, 10969 Berlin (DE)
(72) Erfinder: Flock, Romanus, 10969 Berlin (DE)
(86) Internationale Anmeldenummer: EP9802084
(87) Internationale Veröffentlichungsnummer: WO98046495

(56) Entgegenhaltungen:
- EP-A- 0 540 184
- WO-A-85/04849
- WO-A-92/20595
- WO-A-96/29262
- DE-A- 3 939 679
- DE-U- 29 605 103

## Beschreibung

Als nächstliegender Stand der Technik wird das Dokument **WO 92/20595** angesehen. Es beschreibt eine runde Kondomverpackung die durch Knicken der Verpackung mit drei Fingern der gleichen Hand geöffnet werden kann. Dies wird durch Sollaufreißlinien ermöglicht.

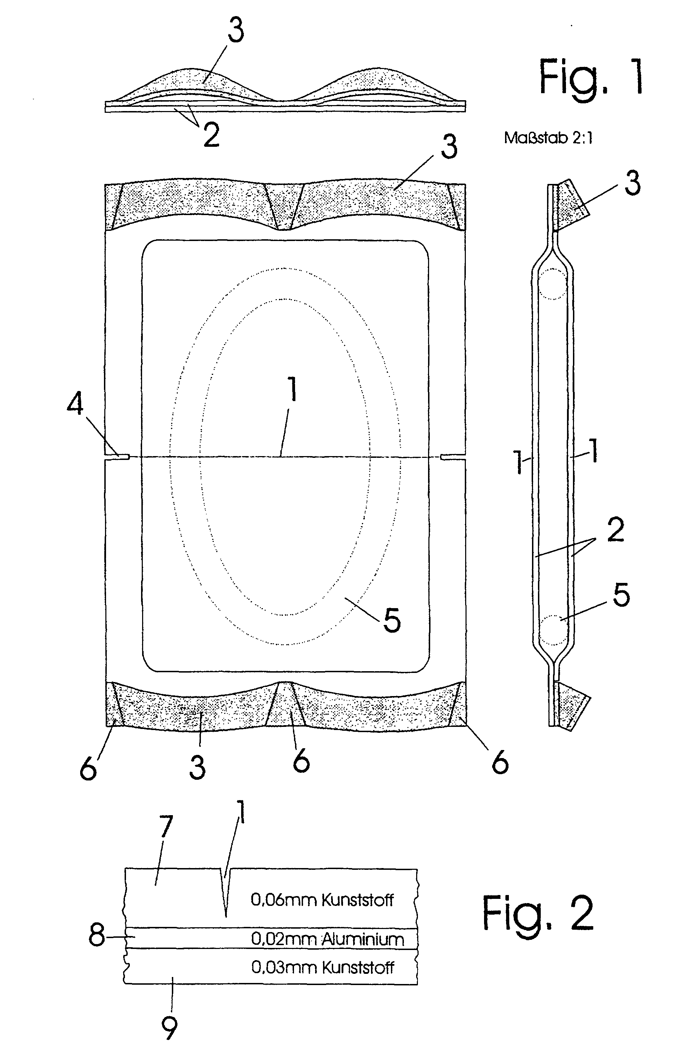

Handelsübliche Kondomverpackungen (2) bestehen aus Aluminium-Kunststoff-Verbundfolien (Fig. 2). Die Hauptfunktion wird durch das Aluminium erfüllt. Es schützt das Kondom vor Luft und Licht. Aluminium alleine würde aber sehr leicht zerreißen. Um dem entgegen zu wirken, ist die ca. 0,02 mm dicke Aluminiumfolie (8) auf der Innenseite mit einem ca. 0,03 mm dicken verschweißbaren Kunststoff (9) beschichtet. Die äußere Aluminiumseite ist mit einem etwa 0,06 mm dicken Schutzkunststoff (7) überzogen, der die Aluminiumfolie (8) vor Beschädigung schützen soll. Durch diese beidseitige Kunststoffbeschichtung wird eine hohe Festigkeit erreicht, was einen guten Schutz gewährleistet. Das Kondom ist zwischen zwei dieser Verbundfolien (2) eingeschweißt.

Diese hohe Festigkeit ist beim Öffnen nachteilig. Öffnen wird erst durch Einkerbungen am Rand der Verpackung möglich. Dazu ist die Schutzverpackung mit zwei Händen an einer Einkerbung mit einer gegenläufigen Bewegung aufzureißen.

Es stellt ein noch größeres Problem dar, daß nirgends zu erkennen ist (selbst eine aufgedruckte Nummer auf der Folie gibt keinen Hinweis) wie herum man nach dem Öffnen das Kondom in der Hand hat. Um ein Kondom überziehen zu können, muß das Abrollen von der richtigen Seite begonnen werden. Versehentliches Verdrehen führt dazu, daß das Kondom nur schwer abgerollt, und im ungünstigsten Fall (Kontaminierung mit Sperma) nicht mehr benutzt werden kann. Ein "Erfühlen" der richtigen Lage ist sehr schwierig, da das Kondom aus sehr dünnem Latex ist und eben ja nicht spürbar sein soll. Eine Sichtprüfung und damit ein Lichteinschalten ist nahezu unumgänglich. Um sicher zu gehen, daß der Abrollvorgang richtig begonnen werden kann, sollte das Kondom etwas abgerollt werden (Empfehlung von Herstellern).

Es stellt sich auch das Problem der Luft im Kondom. Man muß mit zwei Finger das Reservoir an der Spitze des Kondoms zusammendrücken und mit der anderen Hand abrollen.

Alles in allem ist das Überziehen von Kondomen aufwendig, zeitraubend, mitunter ärgerlich.

### Problemlösung:

Eine Lösung all dieser Problem wird durch nachfolgend beschriebene Kondomverpackung (2) behoben. Dadurch wird ein einhändiges, sehr schnelles, weniger als fünf Sekunden dauerndes, Überziehen des Kondoms, auch im Dunkeln, möglich und zwar so, daß sich keinerlei Luft mehr innerhalb des Kondoms befindet.

### Beschreibung:

Die technische Lösung besteht aus dem Anritzen (1) der Verbundfolie (2), dem Einkerben am Ende des Anrisses (4) und dem Anbringen von Schlaufen (3) zum Erkennen der richtigen Lage des Kondoms (5) und dem Aufreißen der Verpackung mit dem Daumen.

Mit einem quer über die gesamte Breite führenden Anriß (1) beider Folien (2) der äußeren Kunststoffbeschichtung (7) (das Aluminium (8) muß aus Haltbarkeitsgründen unversehrt bleiben) und einer Einkerbung (4) am Ende des Anrisses (1), wird es möglich, die Verpackung sehr viel leichter aufzureißen. Und zwar derart, daß die Folie (2) nicht unbedingt mit einer scherenartigen, gegenläufigen Bewegung zweihändig aufgerissen werden muß, sondern durch ein festes in die Hand Schließen, fängt die Hülle (2) an aufzureißen und läßt sich nun mit einer senkrechten Aufwärtsbewegung ohne großen Kraftaufwand mit dem Daumen öffnen.

Um den Daumen einen Halt zu geben, und vor allem auch, um die richtige Lage des Kondoms (5) durch Ertasten erkennen zu können, sind zwei Streifen aus geeignetem Material(2) oben und unten quer an der Rand der Verpackung an drei bzw. zwei Stellen (6), bei schmaleren Kondomverpackungen (Format ca. 26x64 mm), angeschweißt. Dadurch entstehen jeweils oben und unten zwei gleiche bzw. eine, der Daumenkuppe angepaßte, Schlaufen (3). Das ist produktionstechnisch genau so leicht zu realisieren wie das Anbringen einer Schlaufe (3), hat aber den Vorteil, daß die Verpackung (2) von Rechts- und Linkshändern, unabhängig davon ob das verpackte Kondom auf dem Kopf steht, oder nicht, in gleicher Weise geöffnet werden kann.

Nach dem Öffnen befindet sich nun das Kondom (5) in der halben Verpackung, festgehalten mit Mittel-, Ring- und kleinem Finger und kann nun mit Daumen und Zeigefinger übergestreift werden. Dies geschieht dadurch, daß man das halbe aus der Verpackung herausragenden Kondom (5) auf den steifen Penis auflegt, mit dem Daumen und Zeigefinger festhält, die sich noch in der Hand befindlichen Verpackungshälfte von dem Kondom (5) ab streift, und dann das Kondom (5) abrollt. Dieser Vorgang des einhändigen Überstreifens erfordert etwas Übung, ist aber prinzipiell sehr leicht machbar und besteht im Wesentlichen aus nur drei Handgriffen:
Mit dem Daumen aufreißen, richtig ansetzen und abrollen.
Bei den jetzigen Kondomen sind viele Handgriffe nötig, die zum Teil zwei Hände benötigen.

### Zusammenfassung:

Die Kondomverpackung (2) läßt sich mit einer Daumenaufwärtsbewegung leicht öffnen. Das Kondom (5) steht denn in eindeutig richtiger Lage zur Verfügung und kann sofort ohne Zuhilfenahme einer zweiten Hand in wenigen Sekunden übergestreift werden, auch im Dunkeln.

### Produktionsrelevante Komponenten:

Eine wichtige Komponente bei der Entwicklung einer leicht handhabbaren Kondomverpackung, war eine in bestehende Produktionsverfahren integrierbare Lösung.
Das ist gewährleistet, da erstens die bereits verwendeten Materialien bei gleichbleibender Größe weiter verwendet werden können. Zweitens sind die Zusatzwerkzeuge so gering dimensioniert, daß sie leicht in bestehende Lücken in der Produktionsanlage eingebaut werden können.

Das Anritzen der Verpackungsfolie (2) läßt sich realisieren, indem man eine handelsübliche Teppichmesserklinge mit einer Vorrichtung zur vertikaler Führung mit Micrometerschraube, an der entsprechenden Stelle in das laufende Folienband absenkt. Nach dem Berühren der Folienoberfläche wird die Klinge um weitere ein bis zwei Hundertstel Millimeter abgesenkt.

Das Anbringen der Schlaufen (3) funktioniert derart, daß eine der beiden Verpackungsfolien vor dem Einschweißen des Kondoms an den Schlaufenanschweißstellen (6) ausgeklinkt werden muß, dann kann der Kondomeinschweißvorgang wie gehabt fortgesetzt werden. Wenn dann das Kondom verpackt ist, bleiben oben und unten am Rand jeweils drei bzw. zwei der Ausklinkungen (6) entsprechende Flächen mit verschweißbarer Kunststoffbeschichtung frei, an die dann mit entsprechenden Werkzeugen die Folienstreifen angeschweißt werden können, um die Schlaufen (3) zu bilden. Dies funktioniert, weil der Spezialkunststoff nur mit sich selbst verschweißbar ist. Daß vor den Anbringen auf die freien Stellen eine Druck- und Wärmeeinwirkung stattfand, stört weder den Einschweißvorgang selber, noch verhindert es, daß die Schlaufen (3) sich später mit hoher Festigkeit befestigen lassen. Ein direktes Anschweißen an den äußeren Schutzkunststoff ist nicht möglich, da dieser sich trotz Druck und Wärme mit Nichts, auch nicht mit sich selbst, verbindet.

## Patentansprüche

1. Kondomverpackung, bestehend aus zwei zusammen geklappten und angeritzten Folienhälften(2) oder zwei übereinander gelegten, ebenfalls angeritzte Folienteile(2), die an den Rändern miteinander verbunden sind und zwischen sich das Kondom(5) aufnehmen,
**dadurch gekennzeichnet,**
**daß** wenigstens eine Schlaufe(5), Tasche oder ähnliches als Fingereinschub angebracht ist.

2. Kondomverpackung nach Anspruch 1,
**dadurch gekennzeichnet,**
**daß** wenigstens eine der beiden Folienteile oder -hälfte(2) wenigstens eine Aussparung(6) aufweist, an deren Stelle, nach dem Verpacken des Kondoms, Schlaufen(3) oder Taschen angebracht werden können.

3. Kondomverpackung nach Anspruch 1,
**dadurch gekennzeichnet,**
**daß** sich die zichtige Lage des Kondoms in der Verpackung nach den Schlaufen oder Taschen richtet.

## Claims

1. A condom packaging consisting of two folded- together and scored foil halves (2) or two superposed and likewise scored foil parts (2) which are connected to each other at the edges and receive the condom (5) between them, wherein at least one loop (5), pocket or the like is attached as a finger-hold.

2. The condom packaging as claimed in claim 1, wherein at least one of the two foil parts or halves (2) possesses at least one cut-out (6) at the location of which loops (3) or pockets can be attached after the packaging of the condom.

3. The condom packaging as claimed in claim 1, wherein
the wreit position of the condom in the packaging is determined by the loops or pockets.

## Revendications

1. Emballage de préservatif composé de deux demi-feuilles pliées ensemble à fente (2) ou deux pièces de feuille superposées, également à fente (2), reliées l'une à l'autre sur les bords et entre lesquelles le préservatif est placé (5),
**caractérisé par le fait que**
qu'il comporte au moins une bride (5), une poche ou un élément similaire pour insérer le doigt.

2. Emballage de préservatif selon la spécification 1,
**caractérisé par le fait qu'**
au moins l'une des deux feuilles ou une demi-feuille (2) comporte au moins une réserve (6) laquelle servira après l'emballage du préservatif, à placer des brides (3) ou des poches.

3. Emballage de préservatif selon la spécification 1,
**caractérisé par le fait que**
la position correcte du préservatif dans l'emballage est dirigée vers les brides ou les poches.
